# EUROPEAN PATENT APPLICATION

(11) **EP 2 446 920 A1**
(43) Date of publication of application: **02.05.2012**
(21) Application number: 11180500.8
(22) Date of filing: 08.09.2011
(51) Int. Cl.: A61N 1/05

(54) **Implantable leads having coiled conductors to reduce rf-induced current**

(30) Priority: 26.10.2010 US 912563
(71) Applicant: PACESETTER, INC., Sylmar, CA 91392-9221 (US)
(72) Inventor: Kampa, Greg, Castaic, CA California 91384 (US); Doan, Phong D., Stevenson Ranch, CA California 91381 (US)
(74) Representative: Noble, Nicholas

(57) **Abstract**

An implantable lead assembly includes an outer jacket, first and second coiled conductors, and first and second electrodes. The outer jacket is elongated along a center axis and includes a body that radially extends between opposite interior and exterior surfaces. The interior surface defines an elongated lumen. The coiled conductors are held within the body of the outer jacket between the interior and exterior surfaces of the outer jacket and are wrapped around the lumen and the center axis. The electrodes are coupled with the outer jacket and electrically coupled to the coiled conductors in order to at least one of deliver stimulus pulses or sense electrical activity.

## Description

### FIELD OF THE INVENTION

One or more embodiments of the subject matter described herein generally relate to leads that are compatible with magnetic resonance imaging ("MRI") systems.

### BACKGROUND OF THE INVENTION

Implantable electrical medical devices include, but are not limited, to electrocardiographs ("ECGs"), electroencephalographs ("EEGs"), squid magnetometers, implantable pacemakers, implantable cardioverter-defibrillators ("ICDs"), neurostimulators, electrophysiology ("EP") mapping and radio frequency ("RF") ablation systems, and the like (hereafter generally "implantable medical devices" or "IMDs"). IMDs commonly employ one or more leads that receive or deliver voltage, current or other electromagnetic pulses from or to an organ or its surrounding tissue for diagnostic or therapeutic purposes. The leads include bare or insulated coiled wire forming one or more tightly wound solenoid-like structures along the shafts. These tightly wound coils facilitate torque transfer, prevent "buckling" and allow the conduction of electrical signals to and from the proximal (system) end to the distal (patient) end of the lead.

The lead may represent a catheter, an ICD lead, a neurostimulation lead, a pacemaker lead, and the like. When exposed to electromagnetic fields, such as for example those present in MRI systems, the magnetic fields of the MRI systems may induce undesired currents and or voltages in the coils of the leads (referred to as "RF-induced current"). The wires or coils are joined with electrodes of the leads, which may be in contact with surrounding blood or tissue. The RF-induced current interacts with the surrounding blood and tissue and may cause unwanted tissue heating, nerve stimulation, or other negative effects resulting in erroneous diagnosis or therapy delivery.

One approach to reducing RF-induced current in a lead is to provide an RF "choke" in the lead. The choke suppresses RF-induced current from propagating through the lead to the electrodes of the lead. Some RF chokes may include co-radial regions of coils. The RF-induced currents in the two coils can have opposing polarities that cancel each other out to reduce the RF-induced currents form exiting through the electrodes.

The efficiency of some known RF chokes in the leads is limited by the mutual inductance of the coils in the leads. The mutual inductance of the coils represents the ability of one current-carrying coil to induce a current in the other coil. The mutual inductance of the coils can be limited due to the tortuous paths that the leads and coils take through the body of a patient. Increased separation between the coils can reduce the mutual inductance of the coils and thereby reduce the efficiencies of the RF chokes.

A need remains for an improved MRI compatible lead that addresses the above problems and other issues that will be apparent from the following discussion and figures.

### BRIEF SUMMARY OF THE INVENTION

In one embodiment, an implantable lead assembly is provided. The lead assembly includes an outer jacket, first and second coiled conductors, and first and second electrodes. The outer jacket is elongated along a center axis and includes a body that radially extends between opposite interior and exterior surfaces. The interior surface defines an elongated lumen. The coiled conductors are held within the body of the outer jacket between the interior and exterior surfaces of the outer jacket and are wrapped around the lumen and the center axis. The electrodes are coupled with the outer jacket and electrically coupled to the coiled conductors in order to at least one of deliver stimulus pulses or sense electrical activity.

In another embodiment, another implantable lead assembly is provided. The lead assembly includes an elongated outer jacket, first and second coiled conductors, and first and second electrodes. The outer jacket extends along a center axis and has a body that is configured to be implanted in a patient. The coiled conductors are molded within the body of the outer jacket, extend along the center axis, and are wound around the center axis. The first coiled conductor is radially disposed farther from the center axis than the second coiled conductor. The electrodes are coupled with the outer jacket and electrically coupled to the coiled conductors.

In another embodiment, a method for providing an implantable lead assembly is provided. The method includes providing first and second coiled conductors that wrap around a center axis. The first and second coiled conductors are enclosed in insulative layers that engage each other while electrically separating the first and second coiled conductors. The method also includes molding an elongated outer jacket around the first and second coiled conductors such that the first and second coiled conductors are encapsulated in the outer jacket and electrically coupling first and second electrodes with the first and second coiled conductors, respectively.

### BRIEF DESCRIPTION OF THE DRAWINGS

The drawings illustrate generally, by way of example, but not by way of limitation, various embodiments discussed in the present document.

Figure 1 illustrates an implantable medical system including a lead assembly formed in accordance with one embodiment.

Figure 2 illustrates an implantable medical system including the lead assembly shown in Figure 1 formed in accordance with another embodiment.

Figure 3 illustrates the lead assembly shown in Figure 1 in accordance with one embodiment.

Figure 4 is a perspective view of coiled conductors of the lead assembly (shown in Figure 1) in accordance with one embodiment.

Figure 5 is a radial cross-sectional view of the lead assembly along line A-A in Figure 3 in accordance with one embodiment.

Figure 6 is an axial cross-sectional view of the lead assembly along line B-B in Figure 3 in accordance with one embodiment.

Figure 7 is a radial cross-sectional view of the lead assembly along line A-A in Figure 3 in accordance with another embodiment.

Figure 8 is an axial cross-sectional view of the lead assembly along line B-B in Figure 3 in accordance with the embodiment shown in Figure 7.

Figure 9 is an axial cross-sectional view of the lead assembly along line B-B in Figure 3 in accordance with another embodiment.

Figure 10 is a flowchart for a method of manufacturing a lead assembly in accordance with one embodiment.

Figure 11 is an axial cross-sectional view of the lead assembly along line B-B in Figure 3 in accordance with another embodiment.

### DETAILED DESCRIPTION OF THE INVENTION

In the following detailed description, reference is made to the accompanying drawings which form a part hereof, and in which are shown by way of illustration specific embodiments in which the present invention may be practiced. These embodiments, which are also referred to herein as "examples," are described in sufficient detail to enable those skilled in the art to practice the subject matter described herein. It is to be understood that the embodiments may be combined or that other embodiments may be utilized, and that structural, logical, and electrical variations may be made without departing from the scope of the presently described subject matter. For example, embodiments may be used with a variety of medical devices, such as a pacemaker, a cardioverter, a defibrillator, a neurological stimulator, and the like. The following detailed description is, therefore, not to be taken in a limiting sense, and the scope of various embodiments of the presently described subject matter is defined by the appended claims and their equivalents. In this document, the terms "a" or "an" are used to include one or more than one. In this document, the term "or" is used to refer to a nonexclusive "or," unless otherwise indicated.

In accordance with certain embodiments, implantable lead assemblies are provided that include coiled conductors extending through the lead assemblies to electrodes. The lead assembly may be electrically coupled with an implantable cardiac device and implanted into a patient, such as the heart of the patient, to deliver stimulus pulses to one or more chambers of the heat and/or to sense cardiac signals of the patient's heart. Alternatively, the lead assembly may be electrically coupled with a neurological stimulator and implanted into a nervous system of the patient, such as the patient's spine, to deliver stimulus pulses to the nervous system. The coiled conductors, if the lead assembly are arranged and/or positioned within the lead assembly to reduce or eliminate the flow of current that is induced in the coiled conductors when the lead assembly is exposed to a relatively strong magnetic field, such as the magnetic fields generated by MRI systems.

The coiled conductors may allow a greater number of electrodes to be provided on the lead assembly. For example, the lead assembly can include three or more electrodes that are electrically coupled with different coiled conductors in the lead assembly. The coiled conductors may be utilized to independently supply stimulus pulses through the different electrodes and/or independently sense cardiac signals using the different electrodes.

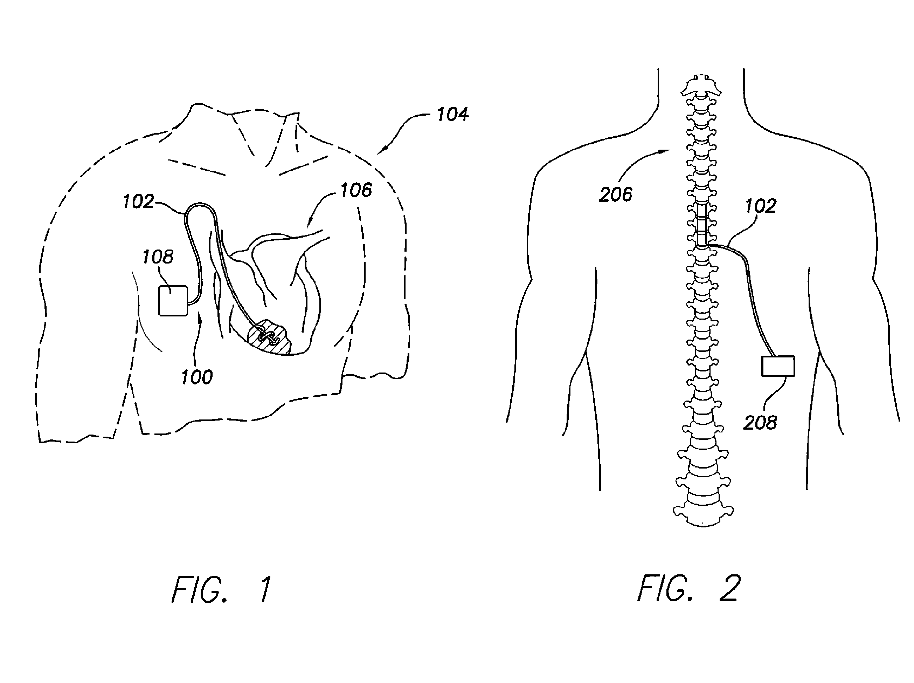

Figure 1 illustrates an implantable medical system 100 including a lead assembly 102 formed in accordance with one embodiment. Figure 1 depicts a chest cavity 104 of a human patient in phantom, and a heart 106 within the chest cavity 104. The medical system 100 includes an implantable medical device (IMD) 108, such as a pacemaker, and the lead assembly 102, which are both implanted in the chest cavity 104. Optionally, the medical device 108 and/or lead assembly 102 may be implanted in a position other than the positions shown in Figure 1. The medical system 100 may be a bipolar device with the lead assembly 102 being a bipolar pacing and sensing lead having two electrodes disposed in one or more chambers of the heart 106. The medical system 100 uses the lead assembly 102 to deliver stimulus pulses to the heart 106 and/or sense cardiac signals of the heart 106. Alternatively, the medical system 100 may be a multi-polar device with the lead assembly 102 being a multi-polar lead having three or more electrodes used to deliver stimulus pulses and/or sense cardiac signals. In another example, the medical system_100 may be a cardiac resynchronization therapy defibrillator or device with the lead assembly 102 having electrodes that deliver stimulus pulses to the heart 106.

Figure 2 illustrates an implantable medical system 200 including the lead assembly 102 formed in accordance with another embodiment. Figure 2 partially depicts a spine 206 in a nervous system of a human patient. The medical system 200 includes a neurological stimulation device 208 and the lead assembly 102. The stimulation device 208 is implanted in the patient with the lead assembly 102 inserted into the spine 206. Optionally, the stimulation device 208 and/or lead assembly 102 may be implanted in a position other than the position shown in Figure 2. The stimulation device 208 may provide spinal cord stimulation (SCS) to reduce pain in the patient. For example, the stimulation device 208 may apply stimulus pulses through the lead assembly 102 to the spine 102 to stimulate the spinal cord or other parts of the central nervous system.

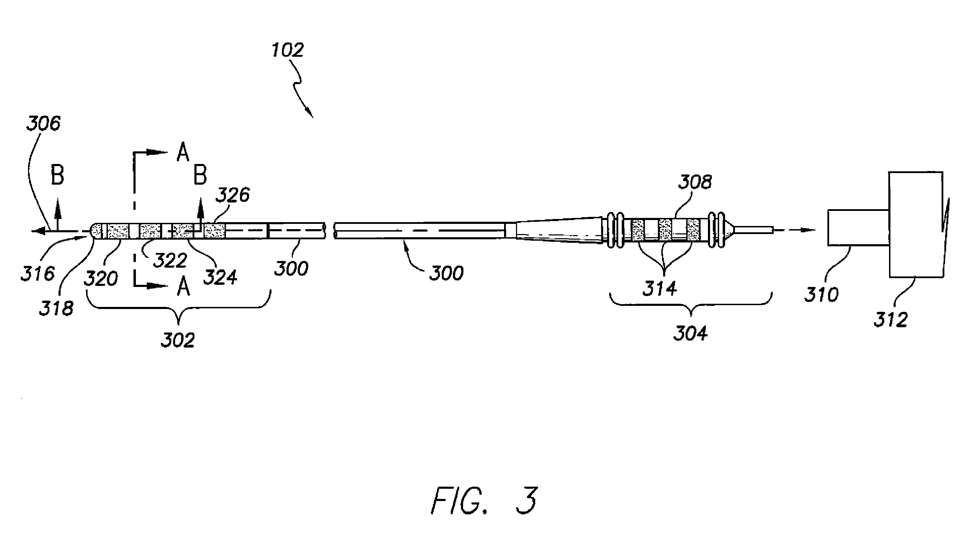

Figure 3 illustrates the lead assembly 102 as having an elongated lead body 300 extending between a distal end portion 302 and a proximal end portion 304 in accordance with one embodiment. The lead body 300 has a length that extends along a center axis 306 between the distal and proximal end portions 302, 304. The term center axis 306 encompasses both linear and nonlinear axes. For example, the center axis 306 of the lead body 300 may extend along a curved or tortuous path that changes as the lead body 300 is flexed, bent, and otherwise manipulated.

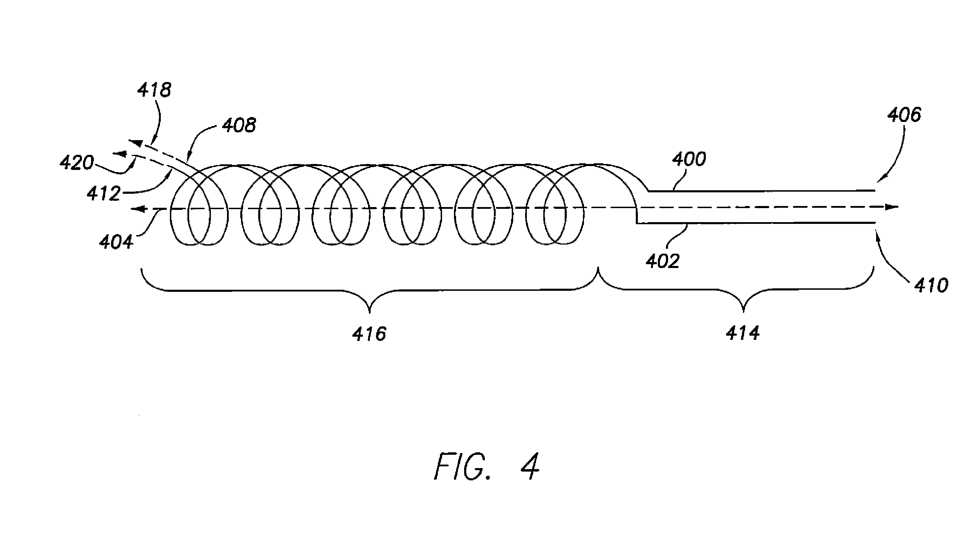

A connector subassembly 308 is provided at the proximal end portion 304 of the lead body 300. The connector subassembly 308 mates with a receiving connector 310 of a medical device 312, such as the IMD 108 (shown in Figure 1) or the stimulation device 208 (shown in Figure 2). The connector subassembly 308 includes electrical terminals 314 connected to coiled conductors 400, 402 (shown in Figure 4), such as pacing and sensing electrical conductors, disposed within the lead body 300.

A header subassembly 316 is provided at the distal end portion 302 of the lead body 300. The header subassembly 316 includes a tip electrode 318 at the distal end portion 302 and several ring electrodes 320, 322, 324, 326 proximate to the distal end portion 302. While four ring electrodes 320, 322, 324, 326 are shown, alternatively the lead body 300 may include a smaller or larger number of ring electrodes 320, 322, 324, 326. The electrode 318, 320, 322, 324, 326 sense electrical activity, such as cardiac signals of the heart 106 (shown in Figure 1), and/or deliver stimulus pulses, such as pacing pulses to the heart 106 or electric pulses to the spine 206 (shown in Figure 2).

Figure 4 is a perspective view of coiled conductors 400, 402 that are disposed within the lead assembly 102 (shown in Figure 1) in accordance with one embodiment. The coiled conductor 400 is elongated between an interface end 406 and an opposite distal end 408 and the coiled conductor 402 is elongated between an interface end 410 and an opposite distal end 412. The interface ends 406, 410 may be coupled with the terminals 314 (shown in Figure 3) such that the coiled conductors 400, 402 are electrically coupled with the medical device 312 (shown in Figure 3) when the connector subassembly 308 (shown in Figure 3) of the lead assembly 102 mates with the receiving connector 310 (shown in Figure 3) of the medical device 312.

In the illustrated embodiment, the coiled conductors 400, 402 include linear portions 414 and coiled portions 416. The linear portions 414 may extend through the proximal end portion 304 (shown in Figure 3) of the lead assembly 102 (shown in Figure 1) while the coiled portions 416 extend from the proximal end portion 304 of the lead assembly 102 to the distal end portion 302 (shown in Figure 3) of the lead assembly 102. Alternatively, the coiled conductors 400, 402 may include only the coiled portions 416 throughout all or substantially all of the length of the coiled conductors 400, 402. In another embodiment, the linear portions 414 may extend beyond the proximal end portion 304.

The coiled conductors 400, 402 are conductive bodies that are wrapped or coiled around a center axis 404. The conductive bodies extend along center axes 418, 420 that follow separate helical or coiled paths in the coiled portions 416. Each of the coiled conductors 400, 402 may be formed from a single conductive body, such as a wire, or from multiple filaments or strands, such as a filar. The coiled conductors 400, 402 convey electric signals, such as sensed cardiac signals of the heart 106 (shown in Figure 1), and/or transmit stimulus pulses, such as shocking pulses or pacing pulses to the heart 106 or electric pulses to the spine 206 (shown in Figure 2). The electric signals and/or pulses may be supplied from the medical device 312 (shown in Figure 3) to the heart 106 or spine 206 through the coiled conductors 400, 402.

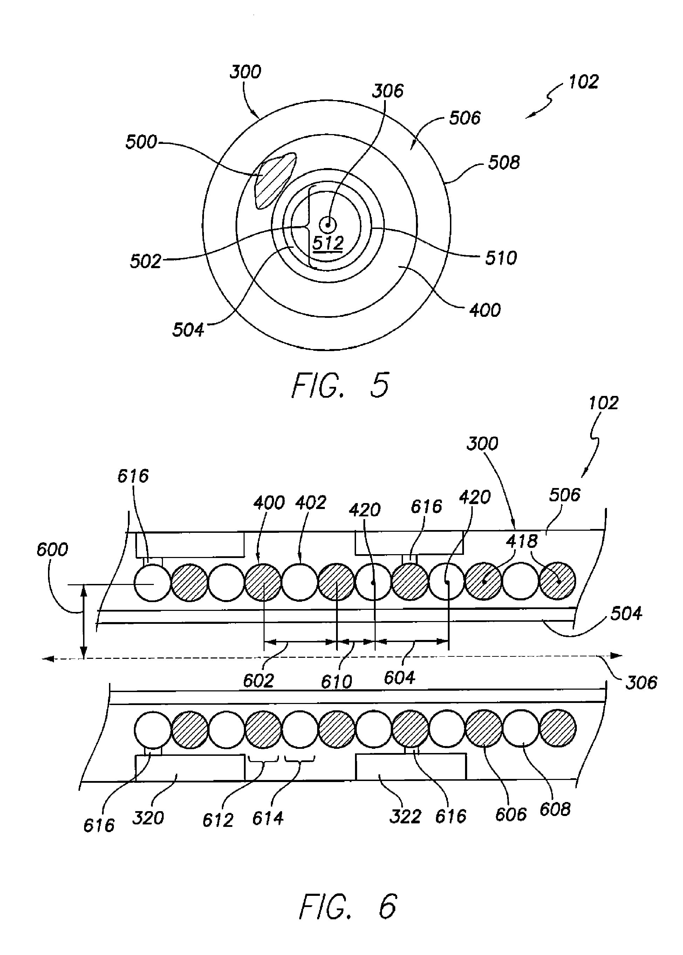

Figure 5 is a radial cross-sectional view of the lead assembly 102 along line A-A in Figure 3 in accordance with one embodiment. The radial cross-sectional view is a view across the center axis 306 of the lead assembly 102 such that the center axis 306 is perpendicular to the plane of view. The body 300 of the lead assembly 102 is symmetric and includes an outer jacket 506 that radially extends between opposite exterior and interior surfaces 508, 510. The outer jacket 506 may be a continuous body that radially extends between the exterior and interior surfaces 508, 510. For example, the coiled conductors 400, 402 may be encapsulated within the outer jacket 506 between the exterior and interior surfaces 508, 510. Alternatively, the body 300 may have an asymmetric cross-sectional shape. The coiled conductors 400, 402 are disposed within the outer jacket 506. While only the coiled conductor 400 is visible in Figure 5, the discussion of the coiled conductor 400 also may apply to the coiled conductor 402. An exposed portion 500 of the coiled conductor 400 illustrates where the plane of the radial cross-sectional view shown in Figure 5 extends through the coiled conductor 400.

As shown in Figures 5 and 6, the coiled conductors 400, 402 are embedded within the body of the outer jacket 506 and are spaced apart from the exterior and interior surfaces 508, 510. In one embodiment, the outer jacket 506 is molded over the coiled conductors 400, 402 such that the coiled conductors 400, 402 are enclosed within and held in place by the outer jacket 506. The outer jacket 506 is formed from a suitable insulative, biocompatible, biostable material such as, for example, PEEK (i.e. Polyetheretherketones), silicone rubber, or polyurethane, that may be molded over and around the coiled conductors 400, 402. Alternatively, the coiled conductors 400, 402 may be inserted into interior gaps or channels of the outer jacket 506 that are located between the exterior and interior surfaces 508, 510. The outer jacket 506 holds the coiled conductor 400, 402 close to each other as the lead body 300 bends and flexes.

The outer jacket 506 defines an elongated outer lumen 502 of the body 300. The outer lumen 502 is bounded by the interior surface 510 of the outer jacket 506 and is elongated through the body 300 along the center axis 306. An inner liner 504, such as an interior sheath or tubular layer, is disposed within the outer lumen 502. The inner liner 504 is elongated through the outer lumen 502 along the center axis 306. In the illustrated embodiment, the inner liner 504 engages the interior surface 510 of the outer jacket 506. Alternatively, the inner liner 504 may be separated from the interior surface 510 such that a gap is provided between the outer jacket 506 and the inner liner 504 within the outer lumen 502.

The inner liner 504 surrounds a central lumen 512. The central lumen 512 is bounded by the inner liner 504 and is elongated through the body 300 along the center axis 306. The inner liner 504 is made of a base material which is an insulating, flexible, dielectric material such as polytetrafluoroethylene (PTFE), ethylene tetrafluoroethylene (ETFE), or a silicone based polymer, for example.

The outer and central lumen 502, 512 are arranged concentric with each another and are centered about the center axis 306. Alternatively, one or more of the outer and central lumen 502, 512 may not be centered about the center axis 306. The central lumen 512 provides a space where an additional conductive body, such as a wire or filar, may be provided. The additional conductive body can extend along the length of the lead assembly 102 to an electrode, such as the tip electrode 318 (shown in Figure 3), to sense electrical activity and/or deliver stimulus pulses using the medical device 312 (shown in Figure 3).

Figure 6 is an axial cross-sectional view of the lead assembly 102 along line B-B in Figure 3 in accordance with one embodiment. The axial cross-section is a view along the center axis 306 of the lead assembly 102 such that the center axis 306 is parallel to the plane of view. The embodiment of the lead assembly 102 shown in Figures 5 and 6 may be referred to as a single plane lead assembly.

The lead assembly 102 is referred to as a single plane lead assembly due to the arrangement of the coiled conductors 400, 402. As shown in Figure 6, the coiled conductors 400, 402 helically wrap around the center axis 306 such that the coiled conductors 400, 402 are co-radial, or are disposed approximately equivalent radial distances from the center axis 306. For example, a radial distance 600 between the center axis 306 and the center axes 418, 420 of the coiled conductors 400, 402 is a common distance for both coiled conductors 400, 402. The axial cross-sectional view shows the coiled conductors 400, 402 being in approximately the same planes both above and below the center axis 306. The radial distance 600 may be different in two or more spaced apart locations along the length of the lead assembly 102.

In the illustrated embodiment, the coiled conductors 400, 402 are wrapped around the center axis 306 at equivalent or approximately equivalent pitch dimensions 602, 604. The pitch dimensions 602, 604 represent axial distances between common points in adjacent turns 612, 614 in the coiled conductors 400, 402. For example, the pitch dimension 602 is measured as the distance in a direction that is parallel to the center axis 306 between the center axes 418 of consecutive turns 612 of the coiled conductor 400. The pitch dimension 604 may similarly be measured for the coiled conductor 402. Alternatively, the pitch dimensions 602, 604 may be represented by the number of turns 612, 614 per unit of length along or parallel to the center axis 306 of the coiled conductors 400, 402.

The cross-sectional view of Figure 6 shows the sectioned turns 612, 614 of the coiled conductors 400, 402 in an alternating arrangement. For example, the turns 612, 614 of the coiled conductors 400, 402 alternate such that each turn 612 of the coiled conductor 400 is located between turns 614 of the coiled conductor 402, and vice-versa. In another embodiment, the pitch dimensions 602, 604 of the coiled conductors 400, 402 may differ such that two or more turns 612 of the coiled conductor 400 are disposed between adjacent turns 614 of the coiled conductor 402. For example, two or more adjacent turns 612 of the coiled conductor 400 may be disposed between non-adjacent or spaced apart turns 614 of the coiled conductor 402, and vice-versa.

The coiled conductors 400, 402 are enclosed in insulative layers 606, 608. The insulative layers 606, 608 are dielectric jackets that surround the coiled conductors 400, 402. The insulative layers 606, 608 prohibit direct electrical contact between the coiled conductors 400, 402. For example, the insulative layers 606, 608 prevent a conductive pathway from being formed between adjacent turns 612, 614 of the coiled conductors 400, 402 when the coiled conductors 400, 402 are disposed next to each other. As shown in Figure 6, the insulative layers 606, 608 engage each other as the coiled conductors 400, 402 helically wrap around the center axis 306 adjacent to each other.

The coiled conductors 400, 402 are electrically coupled with the electrodes 320, 322. Alternatively, one or more of the coiled conductors 400, 402 may be electrically coupled with other electrodes 318, 324, 326 (shown in Figure 3). As both coiled conductors 400, 402 are provided in a single plane arrangement in the embodiment shown in Figure 6, the single plane includes multiple coiled conductors 400, 402 joined with multiple electrodes 320, 322.

The coiled conductors 400, 402 can be electrically coupled with the electrodes 320, 322 by exposing the coiled conductors 400, 402 at or near the electrodes 320, 322. For example, portions of the insulative layers 606, 608 may be removed in locations where the coiled conductors 400, 402 are adjacent to, or pass through, the corresponding electrode 322, 320. The insulative layer 608 of the coiled conductor 402 may remain intact when the coiled conductor 402 is near the electrode 322 to prevent the coiled conductor 402 from being electrically coupled with the electrode 322. Similarly, the coiled conductor 402 may be electrically coupled with the electrode 320 by removing a portion of the insulative layer 608 at or near the electrode 320.

Alternatively, a conductive member, such as conductive solder, may be placed between exposed portions of the coiled conductors 400, 402 and the corresponding electrode 322, 320. In another embodiment, the coiled conductors 400, 402 may be crimped to the corresponding electrode 322, 320. The electric coupling between the coiled conductors 400, 402 and the electrodes 322, 320 is represented by electrical interconnects 616 in Figure 6. The interconnect 616 represents a conductive pathway between each coiled conductor 400, 402 and the electrode 322, 320 to which the coiled conductor 400, 402 is coupled, but is not limited to those examples described above. Other methods or components may be used to provide the conductive pathway.

The coiled conductors 400, 402 have mutual inductance characteristics that block the flow of induced current through the coiled conductors 400, 402. The coiled conductors 400, 402 are sufficiently close that electric current flowing through one coiled conductor 400, 402 induces current in the other coiled conductor 400, 402. This induced current is referred to as "conductor induced current." The mutual inductance characteristics represent the magnitude of the conductor induced current flowing through one coiled conductor 400, 402 when current flows through the other coiled conductor 400, 402. Larger mutual inductance characteristics indicate that a larger magnitude of conductor induced current is generated in one coiled conductor 400, 402 by current flowing through the other coiled conductor 400, 402.

When the coiled conductors 400, 402 are exposed to relatively strong magnetic fields, such as those generated by an MRI system working at radio frequencies such as 64MHz and/or 128MHz, RF-induced current may be induced in each of the coiled conductors 400, 402 by the MRI system. The RF-induced current flowing through one coiled conductor 400, 402 may, in turn, create conductor induced current in the other coiled conductor 400, 402. The mutual inductance characteristics of the coiled conductors 400, 402 are sufficiently large that the RF-induced currents generate approximately equal and opposite conductor induced currents in each of the coiled conductors 400, 402. The conductor induced current may cancel out or significantly decrease the RF-induced current in each of the coiled conductors 400, 402.

For example, the MRI system induces a first RF-induced current in the coiled conductor 400 and a second RF-induced current in the coiled conductor 402. The first and second RF-induced currents may have approximately the same magnitudes but opposite polarities. The coiled conductor 400 creates a first conductor induced current in the coiled conductor 402 based on the first RF-induced current. The first conductor induced current in the coiled conductor 402 may have the same or approximately the same energy or magnitude as the second RF-induced current that also is in the coiled conductor 402. The polarities of the first conductor induced current and the second RF-induced current in the coiled conductor 402 can be opposite of each other. As a result, the first conductor induced current cancels out or significantly reduces the second RF-induced current in the coiled conductor 402. The coiled conductor 402 creates a second conductor induced current in the coiled conductor 400 based on the second RF-induced current. The second conductor induced current in the coiled conductor 400 cancels out or significantly reduces the first RF-induced current in the coiled conductor 402. The RF-induced currents are prevented from flowing through the coiled conductors 400, 402 to the electrodes 320, 322. Blocking the RF-induced currents from reaching the electrodes 320, 322 can prevent the electrodes 320, 322 from heating up or being damaged within the patient, and from damaging tissue to which the electrodes 320, 322 are joined.

In one embodiment, the coiled conductors 400, 402 are held in the outer jacket 506 in positions relative to each other that increase the mutual inductance characteristics of the coiled conductors 400, 402. The coiled conductors 400, 402 are held in the outer jacket 506 relative to each other by a pitch dimension 610. The pitch dimension 610 represents the axial distance between common points of adjacent or neighboring turns 612, 614 of the coiled conductors 400, 402. For example, the pitch dimension 610 shown in Figure 6 is the distance between the center axes 418, 420 of adjacent turns 612, 614 of the coiled conductors 400, 402, respectively. The pitch dimension 610 is measured in a direction parallel to the center axis 306.

The mutual inductance characteristics of the coiled conductors 400, 402 are based on the pitch dimension 610 in one embodiment. As the pitch dimension 610 increases, the separation between the coiled conductors 400, 402 increases. The energy or magnitude of the conductor induced current that is created in each of the coiled conductors 400, 402 by RF-induced current flowing through the other of the coiled conductors 400, 402 decreases as the coiled conductors 400, 402 are farther apart. The decrease in energy or magnitude of conductor induced current in the coiled conductors 400, 402 indicates that the mutual inductance characteristics of the coiled conductors 400, 402 are reduced. As a result, the mutual inductance characteristic decreases with increasing pitch dimensions 610. Conversely, a decrease in the pitch dimension 610 occurs when the coiled conductors 400, 402 are moved closer together. The conductor induced currents increase when the coiled conductors 400, 402 are located closer. As a result, the mutual inductance characteristic increases with decreasing pitch dimensions 610.

In the illustrated embodiment, the coiled conductors 400, 402 are held within the outer jacket 506 such that the insulative layers 606, 608 engage each other. For example, the pitch dimension 610 is sufficiently small that the insulative layers 606, 608 of adjacent turns 612, 614 of the coiled conductors 400, 402 contact each other. Such a pitch dimension 610 represents a smaller separation between the coiled conductors 400, 402 and a greater mutual inductance characteristic of the coiled conductors 400, 402 than larger pitch dimensions 610, where the insulative layers 606, 608 of the adjacent turns 612, 614 may be spaced apart.

Both the coiled conductors 400, 402 may helically wrap around the center axis 306 along the length of the body 300 to at least the electrode 320. For example, the coiled conductor 400 may continue to extend along the length of the body 300 toward the proximal end portion 302 (shown in Figure 3) beyond the electrode 322. The coiled conductor 402 may continue to extend along the length of the body 300 beyond the electrode 320. The coiled conductors 400, 402 can extend beyond the electrodes 320, 322 to which each coiled conductor 400, 402 is joined in order to block induced currents from reaching the electrodes 320,322.

If the coiled conductor 400 did not extend beyond the electrode 322, then the coiled conductor 400 may not cancel out or significantly reduce RF-induced current that is induced in the coiled conductor 402 in some portion of the body 300. For example, the coiled conductor 400 would not be located beyond the electrode 322 and the coiled conductor 400 would not generate a conductor induced current in the coiled conductor 402 that cancels out the RF-induced current in the coiled conductor 402 in locations from the electrode 320 to the electrode 322. Alternatively, the coiled conductors 400, 402 may not extend along the length of the body 300 beyond the electrode 320, 322 to which each coiled conductor 400, 402 is coupled.

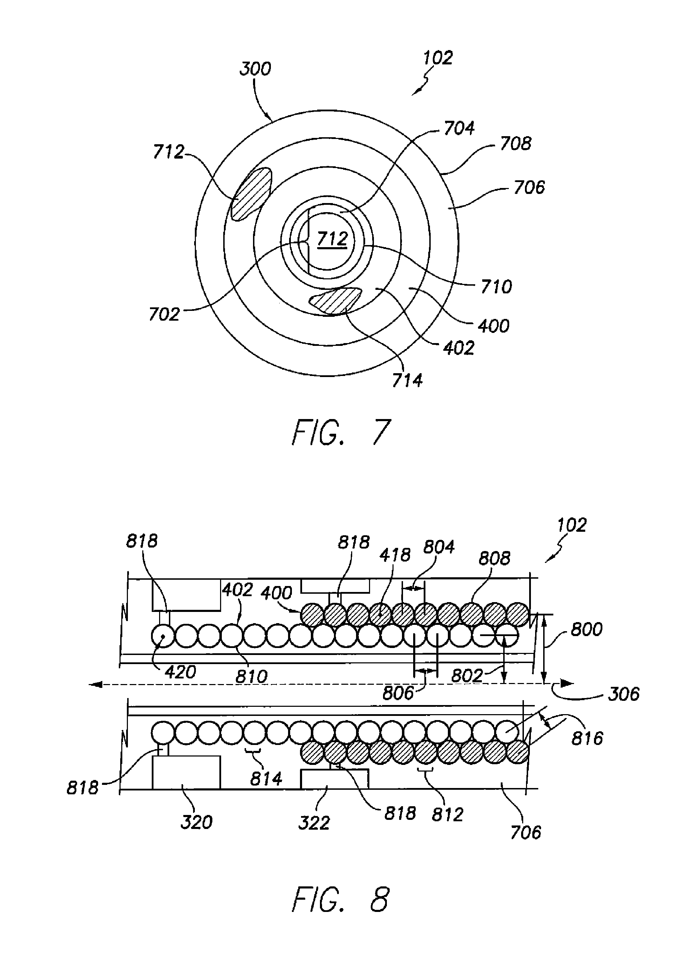

Figure 7 is a radial cross-sectional view of the lead assembly 102 along line A-A in Figure 3 in accordance with another embodiment. The body 300 of the lead assembly 102 shown in Figure 7 is symmetric and includes an outer jacket 706 extending between opposite exterior and interior surfaces 708, 710. The coiled conductors 400, 402 are disposed within the outer jacket 706. Exposed portions 712, 714 of the coiled conductors 400, 402 illustrate where the plane of the radial cross-sectional view shown in Figure 7 extends through the coiled conductors 400, 402.

The outer jacket 706 may be a continuous body that radially extends between the exterior and interior surfaces 508, 510. The coiled conductors 400, 402 may be encapsulated within the outer jacket 706 between the exterior and interior surfaces 708, 710. In one embodiment, the outer jacket 706 is molded over the coiled conductors 400, 402 such that the coiled conductors 400, 402 are enclosed within and held in place by the outer jacket 706. The outer jacket 706 is formed from a suitable insulative, biocompatible, biostable material such as, for example, PEEK, silicone rubber, or polyurethane, that may be molded over and around the coiled conductors 400, 402. Alternatively, the coiled conductors 400, 402 may be inserted into interior gaps or channels of the outer jacket 706.

The outer jacket 706 defines an elongated outer lumen 702 of the body 300 that is bounded by the interior surface 710 of the outer jacket 706. An inner liner 704 is disposed within the outer lumen 702 and engages the interior surface 710 of the outer jacket 706. Alternatively, the inner liner 704 may be separated from the outer jacket 706. The inner liner 704 surrounds a central lumen 712. The inner liner 704 is made of a base material which is an insulating, flexible, dielectric material such as PTFE, ethylene ETFE or a silicone based polymer, for example. The central lumen 712 provides a space where an additional conductive body, such as a wire or filar, may be provided.

Figure 8 is an axial cross-sectional view of the lead assembly 102 along line B-B in Figure 3 in accordance with the embodiment shown in Figure 7. The coiled conductors 400, 402 may be wrapped around the center axis 306 at an equivalent or approximately equivalent pitch dimensions 804, 806. Alternatively, the pitch dimensions 804, 806 may differ from each other.

The embodiment of the lead assembly 102 shown in Figures 7 and 8 may be referred to as a multiple plane lead assembly due to the arrangement of the coiled conductors 400, 402. In contrast to the single plane arrangement of Figures 5 and 6, the coiled conductors 400, 402 helically wrap around the center axis 306 such that the coiled conductors 400, 402 are arranged in different layers that are disposed different radial distances from the center axis 306. For example, the coiled conductor 400 is arranged in an outer layer that is located a radial distance 800 between the center axis 306 and the center axis 418 of the coiled conductor 400. The coiled conductor 402 is arranged in an inner layer that is located a radial distance 802 from the center axis 306. As shown in Figure 8, the radial distance 800 of the outer layer is larger than the radial distance 802 of the inner layer. Consequently, the coiled conductors 400, 402 are not co-radial in the illustrated embodiment. Alternatively, the lead assembly 102 may include three or more concentric layers with each layer including a different single coiled conductor.

The axial cross-sectional view shows sectioned turns 812, 814 of the coiled conductors 400, 402 in a non-alternating arrangement. In contrast to the embodiment shown in Figure 6, each layer of the coiled conductors 400, 402 includes only a single one of the coiled conductors 400, 402. In the embodiment of Figure 6, the single layer included both coiled conductors 400, 402 arranged in an alternating pattern or arrangement.

The coiled conductors 400, 402 are enclosed in insulative layers 808, 810. The insulative layers 808, 810 are dielectric jackets that surround the coiled conductors 400, 402 and prohibit direct electrical contact between the coiled conductors 400, 402. The insulative layers 808, 810 engage each other as the coiled conductors 400, 402 helically wrap around the center axis 306 adjacent to each other. The insulative layers 808, 810 may be deposited on outer surfaces of the coiled conductors 400, 402 to prevent an electric short between adjacent layers of the coiled conductors 400, 402. For example, in order to prevent the insulative layers 808, 810 from being separated or delaminated from the coiled conductors 400, 402, the insulative layers 808, 810 may be deposited onto the coiled conductors 400, 402 after the coiled conductors 400, 402 are formed into the helical shapes shown in Figure 4. Depositing the insulative layers 808, 810 onto the coiled conductors 400, 402 after the coiled conductors 400, 402 are formed into helical shapes rather than prior to forming the coiled conductors 400, 402 into the helical shapes can prevent the insulative layers 808, 810 from separating from the coiled conductors 400, 402.

The thicknesses of the insulative layers 808, 810 may be increased relative to known coiled conductors in leads in order to prevent the insulative layers 808, 810 from being completely removed in adjacent layers. The removal of the insulative layers 808, 810 in adjacent layers may allow the coiled conductors 400, 402 of the adjacent layers to contact each other and form an electrical short therebetween. In one embodiment, the insulative layers 808, 810 are deposited in thicknesses that are at least 1.0 mil (or 0.025 millimeters). Alternatively, the insulative layers 808, 810 may be at least 1.5 mils, 2.0 mils, 2.5 mils or greater in thickness (or 0.038 millimeters, 0.051 millimeters, or 0.064 millimeters). The insulative layers 808, 810 may include or be formed from electrically insulative materials, such as polyurethane, silicone, a mixture of polyurethane and silicon (such as Optim™), ETFE, or PTFE, for example.

In the illustrated embodiment, the turns 814 of the coiled conductor 402 are centered between the turns 812 of the coiled conductor 400. For example, the center axis 420 of coiled conductor 402 is slightly offset in an axial direction that is parallel to the center axis 306 from the center axis 418 of the coiled conductor 400.

The coiled conductors 400, 402 are axially offset from each other such that the insulative layer 808 of each of the turns 812 of the coiled conductor 400 engages the insulative layer 810 of each of the turns 814 of the coiled conductor 402 twice, and vice versa. For example, each turn 812 of the coiled conductor 400 engages two turns 814 of the coiled conductor 402 and two adjacent or neighboring turns 812 of the same coiled conductor 400. Each turn 814 of the coiled conductor 402 engages two turns 812 of the coiled conductor 400 and two adjacent or neighboring turns 814 of the same coiled conductor 402. Alternatively, each turn 812, 814 of a coiled conductor 400, 402 may engage a different number of turns 812, 814 of the same or the other coiled conductor 400, 402.

The coiled conductors 400, 402 are electrically coupled with the electrodes 320, 322. Alternatively, one or more of the coiled conductors 400, 402 may be electrically coupled with other electrodes 318, 324, 326 (shown in Figure 3). The coiled conductors 400, 402 can be electrically coupled with the electrodes 320, 322 by exposing the coiled conductors 400, 402 at or near the electrodes 320, 322. Alternatively, a conductive member, such as conductive solder, may be placed between exposed portions of the coiled conductors 400, 402 and the corresponding electrode 322, 320. In another embodiment, the coiled conductors 400, 402 may be crimped to the corresponding electrode 322, 320. The electric coupling between the coiled conductors 400, 402 and the electrodes 322, 320 is represented by electrical interconnects 818 in Figure 8. The interconnect 818 represents a conductive pathway between each coiled conductor 400, 402 and the electrode 322, 320 to which the coiled conductor 400, 402 is coupled, but is not limited to those examples described above. Other methods or components may be used to provide the conductive pathway.

In contrast to the single plane arrangement of Figure 6, the embodiment shown in Figure 8 includes multiple layers of the coiled conductors 400, 402 joined with multiple electrodes 320, 322. Each electrode 320, 322 is coupled with a different plane of the coiled conductors 400, 402. For example, the electrode 320 is coupled with the coiled conductor 400 in the plane disposed farther from the center axis 306 (e.g., the outer plane) and the electrode 322 is coupled with the coiled conductor 402 in the plane disposed closer to the center axis 306 (e.g., the inner plane).

The coiled conductors 400, 402 are held sufficiently close together by the outer jacket 706 that the coiled conductors 400, 402 cancel out or significantly reduce RF-induced current in the coiled conductors 400, 402. The coiled conductors 400, 402 are held in the outer jacket 706 relative to each other by a pitch dimension 816. The pitch dimension 816 represents the distance between common points of adjacent or neighboring turns of the coiled conductors 400, 402. For example, the pitch dimension 816 may be measured as the distance between the center axes 612, 614 of turns 812, 814 of the coiled conductors 400, 402 that engage each other. The pitch dimension 816 is measured in a direction that is obliquely or perpendicularly oriented with respect to the center axis 306.

The mutual inductance characteristics of the coiled conductors 400, 402 are based on the pitch dimension 816 in one embodiment. As the pitch dimension 816 increases, the mutual inductance characteristics of the coiled conductors 400, 402 decreases. Conversely, as the pitch dimension 816 decreases, the mutual inductance characteristics of the coiled conductors 400, 402 may increase.

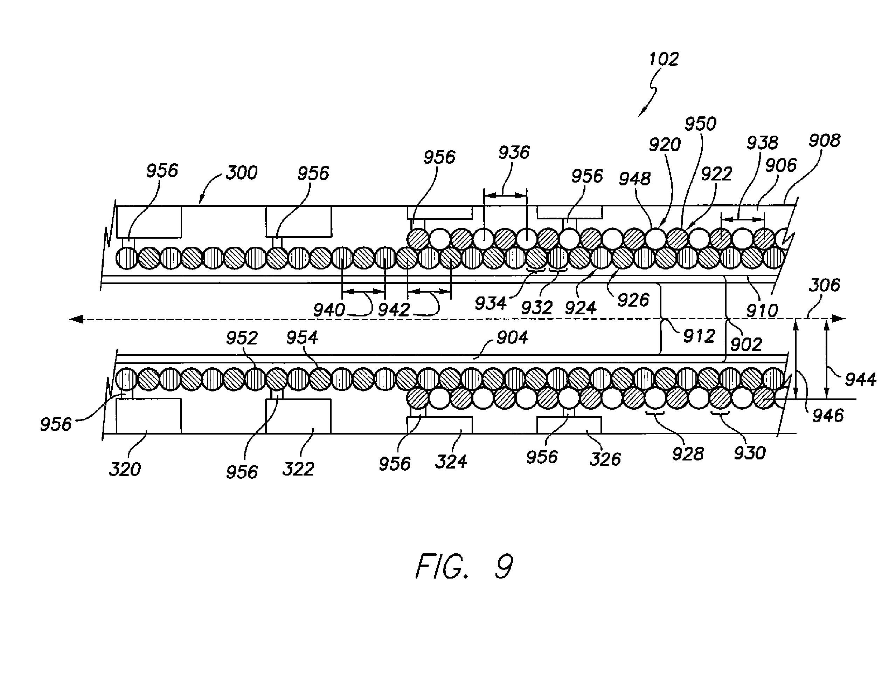

Figure 9 is an axial cross-sectional view of the lead assembly 102 along line B-B in Figure 3 in accordance with another embodiment. The body 300 of the lead assembly 102 shown in Figure 9 is symmetric and includes an outer jacket 906 extending between opposite exterior and interior surfaces 908, 910. The outer jacket 906 defines an elongated outer lumen 902 of the body 300 that is bounded by the interior surface 910 of the outer jacket 906. An inner liner 904 is disposed within the outer lumen 902 and engages the interior surface 910 of the outer jacket 906. Alternatively, the inner liner 904 may be separated from the outer jacket 906. The inner liner 904 surrounds a central lumen 912. The central lumen 912 provides a space where an additional conductive body, such as a wire or filar, may be provided along the center axis 306.

The lead assembly 102 shown in Figure 9 includes four coiled conductors 920, 922, 924, 926. Similar to the coiled conductors 400, 402 (shown in Figure 4), the coiled conductors 920, 922, 924, 926 are disposed within the outer jacket 906. For example, the coiled conductors 920, 922, 924, 926 may molded or otherwise held within the outer jacket 906 between the exterior and interior surfaces 908, 910. The coiled conductors 920, 922, 924, 926 are wrapped around the center axis 306 of the body 300, such as by being helically wrapped around the center axis 306.

The embodiment of the lead assembly 102 shown in Figure 9 may be referred to as a multiple coil, multiple plane lead assembly due to the arrangement of the coiled conductors 920, 922, 924, 926. In contrast to the single plane arrangement of Figures 5 and 6, the coiled conductors 920, 922, 924, 926 helically wrap around the center axis 306 such that the coiled conductors 920, 922, 924, 926 are disposed different radial distances from the center axis 306 and form two separate planes above and below the center axis 306. For example, the coiled conductors 920, 922 are arranged in an outer layer that is located a radial distance 944 from the center axis 306 and the coiled conductors 924, 926 are arranged in an inner layer that is located a radial distance 946 from the center axis 306. As shown in Figure 9, the radial distance 944 of the outer layer is larger than the radial distance 946 of the inner layer.

In contrast to the multiple layer, single coil per layer arrangement shown in Figures 7 and 8, each of the outer and inner layers formed by the coiled conductors 920, 922, 924, 926 include multiple coiled conductors 920, 922, 924, 926. For example, the outer layer includes the coiled conductors 920, 922 while the inner layer includes the coiled conductors 924, 926. The embodiment shown in Figure 9 may be referred to as a multiple layer, multiple coil per layer arrangement. While only two coiled conductors 920, 922, 924, 926 are in each of the outer and inner layers, alternatively a single coiled conductor 920, 922, 924, 926 may be provided in one or more of the layers. Also, while only two layers are formed by the coiled conductors 920, 922, 924, 926, alternatively three or more layers may be formed by the coiled conductors 920, 922, 924, 926. For example, the lead assembly 102 may have three or more layers with each layer including a plurality of coiled conductors.

The coiled conductors 920, 922, 924, 926 include several turns 928, 930, 932, 934 that wrap around the center axis 306. The turns 928, 930, 932, 934 are separated corresponding pitch dimensions 936, 938, 940, 942 in directions that are parallel to the center axis 306. In the illustrated embodiment, the pitch dimensions 936, 938, 940, 942 are equivalent or approximately equivalent. Alternatively, the pitch dimensions 936, 938, 940, 942 may be different for the different layers formed by the coiled conductors 920, 922, 924, 926. For example, the pitch dimensions 936, 938 of the coiled conductors 920, 922 in the outer layer may be approximately the same and the pitch dimensions 940, 942 of the coiled conductors 924, 926 of the inner layer may be approximately the same but different from the pitch dimensions 936, 938 of the outer layer. In another embodiment, the pitch dimensions 936, 938, 940, 942 within the outer or inner layer may be different from each other.

The coiled conductors 920, 922, 924, 926 within each of the outer and inner layers are shown in an alternating arrangement or pattern. For example, the turns 928, 930 of the coiled conductors 920, 922 in the outer layer alternate such that each turn 928 of the coiled conductor 920 is disposed between pairs of the turns 930 of the coiled conductor 922 and each turn 930 of the coiled conductor 922 is located between pairs of the turns 928 of the coiled conductor 920. The turns 932, 934 of the coiled conductors 924, 926 also are arranged in an alternating pattern with each turn 932 of the coiled conductor 924 disposed between pairs of the turns 934 of the coiled conductor 926, and vice-versa. Alternatively, the outer and/or inner layer of the coiled conductors 920, 922, 924, 926 may have a non-alternating arrangement. By way of example only, two or more turns 928, 930, 932, 934 of one or more the coiled conductors 920, 922, 924, 926 may be disposed between adjacent turns 928, 930, 932, 934 of another coiled conductor 920, 922, 924, 926. In another embodiment, one or more of the outer and/or inner layers may have a single coiled conductor 920, 922, 924, 926 or more than three coiled conductors 920, 922, 924, 926.

The coiled conductors 920, 922, 924, 926 are enclosed in insulative layers 948, 950, 952, 954. The insulative layers 948, 950, 952, 954 are dielectric jackets that surround the coiled conductors 920, 922, 924, 926 and prohibit direct electrical contact between the coiled conductors 920, 922, 924, 926. The insulative layers 948, 950, 952, 954 may be deposited on outer surfaces of the coiled conductors 920, 922, 924, 926 to prevent an electric short between adjacent layers of the coiled conductors 920, 922, 924, 926. For example, in order to prevent the insulative layers 948, 950, 952, 954 from being separated or delaminated from the coiled conductors 920, 922, 924, 926, the insulative layers 948, 950, 952, 954 may be deposited onto the coiled conductors 920, 922, 924, 926 after the coiled conductors 920, 922, 924, 926are formed into helical shapes. The thicknesses of the insulative layers 948, 950, 952, 954 may be increased relative to known coiled conductors in leads in order to prevent the insulative layers 948, 950, 952, 954 from being completely removed in adjacent layers. The removal of the insulative layers 948, 950, 952, 954 in adjacent layers may allow the coiled conductors 920, 922, 924, 926 of the adjacent layers to contact each other and form an electrical short therebetween. In one embodiment, the insulative layers 948, 950, 952, 954 are deposited in thicknesses that are at least 1.0 mil (or 0.025 millimeters). Alternatively, the insulative layers 948, 950, 952, 954 may be at least 1.5 mils, 2.0 mils, 2.5 mils or greater in thickness (or 0.038 millimeters, 0.051 millimeters, or 0.064 millimeters). The insulative layers 948, 950, 952, 954 may include or be formed from electrically insulative materials, such as polyurethane, silicone, a mixture of polyurethane and silicon (such as Optim™), ETFE, or PTFE, for example.

The insulative layers 948, 950, 952, 954 engage each other as the coiled conductors 920, 922, 924, 926 helically wrap around the center axis 306 adjacent to each other. The coiled conductors 920, 922, 924, 926 are arranged such that the insulative layers 948, 950, 952, 954 around adjacent turns 928, 930, 932, 934 of the coiled conductors 920, 922, 924, 926 engage each other. For example, the insulative layer 948, 950, 952, 954 of each coiled conductor 920, 922, 924, 926 engages the insulative layers 948, 950, 952, 954 of the other coiled conductors 920, 922, 924, 926. Alternatively, one or more of the insulative layers 948, 950, 952, 954 may engage less than all of the insulative layers 948, 950, 952, 954 of the other coiled conductors 920, 922, 924, 926.

The coiled conductors 920, 922, 924, 926 are electrically coupled with the electrodes 320, 322, 324, 326. Alternatively, one or more of the coiled conductors 920, 922, 924, 926 may be electrically coupled with the electrode 318 (shown in Figure 3). The coiled conductors 920, 922, 924, 926 can be electrically coupled with the electrodes 320, 322, 324, 326 by exposing the coiled conductors 920, 922, 924, 926 at or near the electrodes 320, 322, 324, 326. Alternatively, a conductive member, such as conductive solder, may be placed between exposed portions of the coiled conductors 920, 922, 924, 926 and the corresponding electrode 320, 322, 324, 326. In another embodiment, the coiled conductors 920, 922, 924, 926 may be crimped to the corresponding electrode 320, 322, 324, 326. The electric coupling between the coiled conductors 920, 922, 924, 926 and the electrodes 320, 322, 324, 326 is represented by electrical interconnects 956 in Figure 9. The interconnect 956 represents a conductive pathway between each coiled conductor 920, 922, 924, 926 and the electrode 320, 322, 324, 326 to which the coiled conductor 920, 922, 924, 926 is coupled, but is not limited to those examples described above. Other methods or components may be used to provide the conductive pathway.

In the illustrated embodiment, the electrode 320 is coupled with the coiled conductor 920, the electrode 322 is coupled with the coiled conductor 922, the electrode 324 is coupled with the coiled conductor 924, and the electrode 326 is coupled with the coiled conductor 926.

The coiled conductors 920, 922, 924, 926 are held sufficiently close together by the outer jacket 906 that the coiled conductors 920, 922, 924, 926 cancel out or significantly reduce RF-induced current in the coiled conductors 920, 924, 926, 928. In one embodiment, the coiled conductors 920, 922, 924, 926 in each layer mutually cancel out or significantly reduce the RF-induced current in the coiled conductors 920, 922, 924, 926 of that layer. For example, with respect to the outer layer, the coiled conductors 920, 922 may cancel out or eliminate the RF-induced current in the coiled conductors 920, 922 while the coiled conductors 924, 926 in the inner layer cancel out or eliminate the RF-induced current in the coiled conductors 924, 926. Alternatively, one or more of the coiled conductors 920, 922, 924, 926 in one layer reduces or eliminates the RF-induced current in one or more coiled conductors 920, 922, 924, 926 located in another layer. For example, the coiled conductor 920 and/or 922 can reduce or eliminate RF-induced current in the coiled conductor 924 and/or 926.

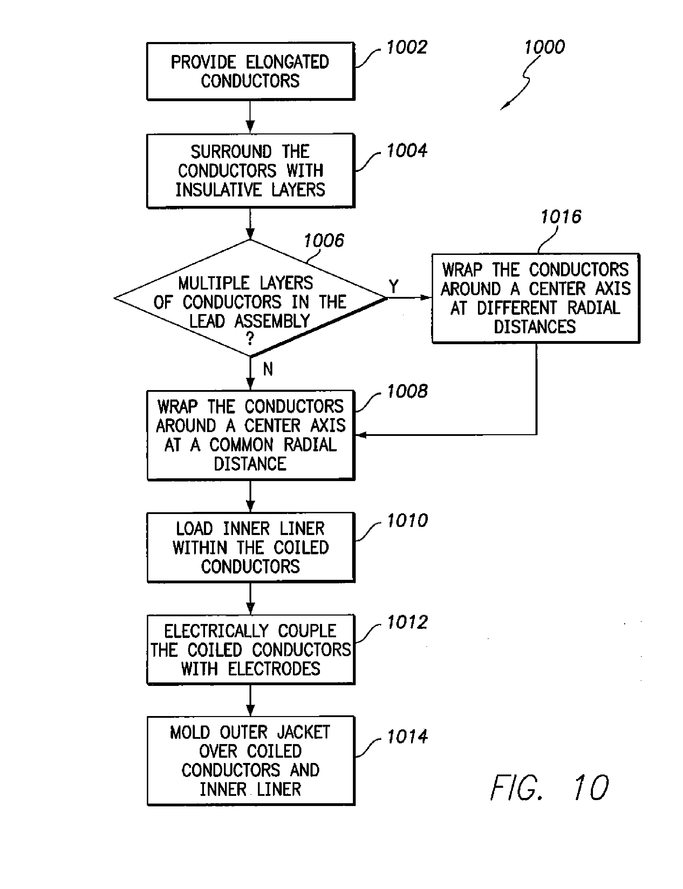

Figure 10 is a flowchart for a method 1000 of manufacturing a lead assembly in accordance with one embodiment. The method 1000 may be used to provide one or more embodiments of the lead assembly 102 (shown in Figure 1) as described herein. For example, the method 1000 can be utilized to provide the single layer, multiple conductors per layer arrangement shown in Figure 6, the multiple layers, single conductor per layer arrangement shown in Figure 8, or the multiple layers, multiple conductors per layer arrangement shown in Figure 9.

At 1002, elongated conductors are provided. The conductors may be elongated conductive bodies such as wires or filars. The lengths of the conductors are sufficiently long that the conductors extend from the proximal end portion 304 (shown in Figure 3) of the lead assembly 102 (shown in Figure 1) to one or more of the electrodes 318, 320, 322, 324 (shown in Figure 3) once the conductors are coiled.

At 1004, the conductors are surrounded by insulative layers. For example, the conductors may be enclosed in dielectric layers that form the insulative layers 606, 608, 808, 810, 948, 950, 952, 954 (shown in Figures 6, 8, and 9). The insulative layers can entirely enclose the conductors. Alternatively, portions of the insulative layers are removed or portions of the conductors are left exposed such that the conductors can be electrically coupled with the terminals 314 (shown in Figure 3) and/or the electrodes 318, 320, 322, 324 (shown in Figure 3). For example, the conductive interconnect 616, 818, 956, 1158 (shown in Figures 6, 8, 9, and 11) may be used to electrically couple the conductors with the electrodes.

At 1006, a determination is made as to whether the lead assembly will include multiple layers of conductors. For example, a determination is made as to whether the lead assembly 102 (shown in Figure 1) will include a single layer of conductors (as shown in Figure 6) or two or more layers of conductors (as shown in Figures 8 and 9). If the lead assembly will include a single layer of conductors, flow of the method 1000 proceeds to 1008. Alternatively, if the lead assembly will include more than one layer of conductors, flow of the method 1000 proceeds to 1016.

At 1008, the conductors are wrapped around a center axis at a common radial distance. For example, the conductors may be helically wrapped around the center axis 306 (shown in Figure 3) in order to form the helically coiled conductors 400, 402 (shown in Figure 4). The conductors can be wrapped around the center axis 306 by winding the conductors around the outer surface of a tube or cylindrical body. In one embodiment, the conductors are wrapped such that the insulative layers of the conductors engage each other. As shown in Figure 6, the insulative layer 606 of the coiled conductor 400 engages adjacent turns 614 of the insulative layer 608 of the coiled conductor 402 and the insulative layer 608 of the coiled conductor 402 engages adjacent turns 612 of the insulative layer 606. The coiled conductors 400, 402 may be wrapped such that the insulative layers 606, 608 engage each other to reduce the pitch dimension 610 (shown in Figure 6) between the coiled conductors 400, 402 and increase a mutual inductance characteristic of the coiled conductors 400, 402.

Alternatively, if flow of the method 1000 proceeds from 1016 to 1016, the conductors are wrapped around a center axis at different radial distances. The conductors may be helically wrapped around the center axis 306 (shown in Figure 3) at different distances from the center axis 306 to form the helically coiled conductors 400, 402 shown in Figure 8 and/or the coiled conductors 920, 922, 924, 926 shown in Figure 9. The conductors are wrapped such that the conductors are separated from the center axis 306 by different distances, thereby forming two or more layers of coiled conductors.

At 1010, an inner liner is loaded within the coiled conductors. For example, the inner liner 504, 704, 904 (shown in Figures 5, 7, and 9) may be inserted within the coiled conductors such that the inner layer 504, 704, 904 is radially disposed between the center axis 306 (shown in Figure 3) and the coiled conductors. The inner liner 504, 704, 904 defines the central lumen 512, 712, 912 (shown in Figures 5, 7, and 9) that extends through the body 300 (shown in Figure 3) of the lead assembly 102. An additional elongated conductor may be placed inside the central lumen 512, 712, 912.

At 1012, the coiled conductors are electrically coupled with one or more electrodes. For example, the coiled conductors 400, 402, 920, 922, 924, 926 (shown in Figures 4 and 9) may be electrically coupled with one or more of the electrodes 318, 320, 322, 324, 326 (shown in Figure 3). The coiled conductors may be electrically coupled with the electrodes by locally removing portions of the insulative layers 606, 608, 808, 810, 948, 950, 952, 954 (shown in Figures 6, 8, and 9) from the coiled conductors. For example, the insulative layer of all or a portion of the coiled conductor that engages, contacts, or extends through an electrode may be removed such that the coiled conductor and the electrode engage each other. The engagement between the coiled conductor and the electrode can electrically couple the coiled conductor and the electrode.

At 1014, an outer jacket is molded over the coiled conductors and the inner liner. The outer jacket may be formed by overmolding a flexible material such as PEEK (i.e. Polyetheretherketones), silicone rubber or polyurethane onto the coiled conductors and inner liner. The outer jacket encapsulates the coiled conductors and the inner liner to enclose the body 300 (shown in Figure 3) of the lead assembly 102. The outer jacket holds the coiled conductors in a relatively close relationship. For example, the coiled conductors may be wound around the center axis 306 (shown in Figure 3) such that the coiled conductors are disposed close to each other and have relatively large mutual inductance characteristics. The outer jacket can be molded around and over the coiled conductors to hold and keep the coiled conductors in a relatively close relationship with each other.

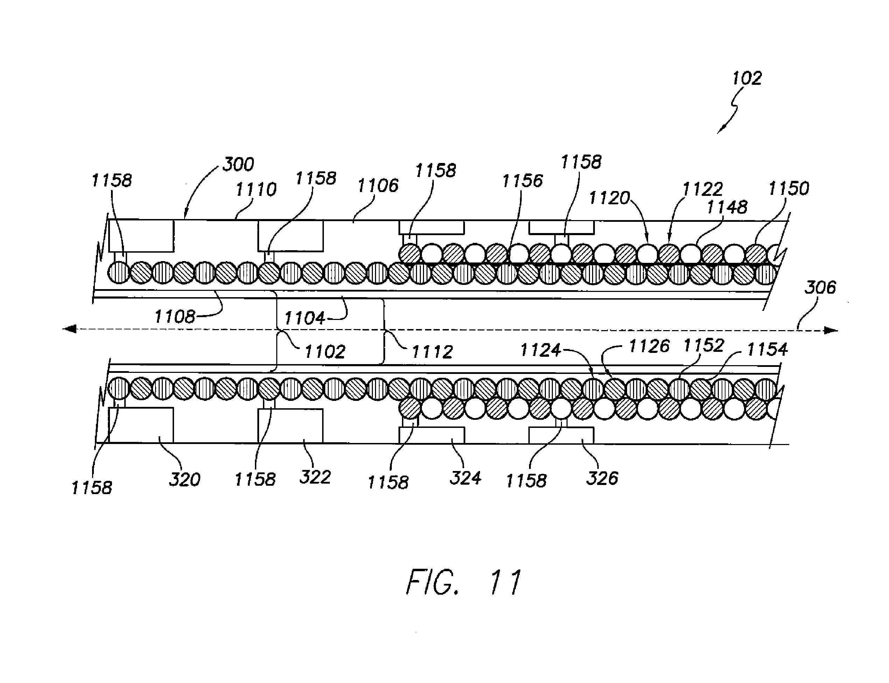

Figure 11 is an axial cross-sectional view of the lead assembly 102 along line B-B in Figure 3 in accordance with another embodiment. The body 300 of the lead assembly 102 shown in Figure 11 is similar to the body 300 of the lead assembly 102 shown in Figure 9. For example, the lead assembly 102 of the embodiment shown in Figure 11 includes an outer jacket 1106 extending between opposite exterior and interior surfaces 1108, 1110. The outer jacket 1106 defines an elongated outer lumen 1102 of the body 300 that is bounded by the interior surface 1110. An inner liner 1104 is disposed within the outer lumen 1102 and surrounds a central lumen 1112.

Four coiled conductors 1120, 1122, 1124, 1126 that are similar to the coiled conductors 920, 922, 924, 926, 928 (shown in Figure 9) are disposed in two layers within the outer jacket 1106. Alternatively, a different number of coiled conductors 1120, 1122, 1124, 1126 and/or a greater number of layers may be provided. The coiled conductors 1120, 1122, 1124, 1126 are enclosed in insulative layers 1148, 1150, 1152, 1154 that are similar to the insulative layers 948, 950, 952, 954 (shown in Figure 9).

The coiled conductors 1120, 1122, 1124, 1126 are electrically coupled with the electrodes 320, 322, 324, 326. Alternatively, one or more of the coiled conductors 1120, 1122, 1124, 1126 may be electrically coupled with the electrode 318 (shown in Figure 3). The coiled conductors 1120, 1122, 1124, 1126 can be electrically coupled with the electrodes 1120, 1122, 1124, 1126 by exposing the coiled conductors 920, 922, 924, 926 at or near the electrodes 1120, 1122, 1124, 1126. Alternatively, a conductive member, such as conductive solder, may be placed between exposed portions of the coiled conductors 920, 922, 924, 926 and the corresponding electrode 1120, 1122, 1124, 1126. In another embodiment, the coiled conductors 920, 922, 924, 926 may be crimped to the corresponding electrode 1120, 1122, 1124, 1126. The electric coupling between the coiled conductors 920, 922, 924, 926 and the electrodes 1120, 1122, 1124, 1126 is represented by electrical interconnects 1158 in Figure 11. The interconnect 1158 represent a conductive pathway between each coiled conductor 1120, 1122, 1124, 1126 and the electrode 320, 322, 324, 326 to which the coiled conductor 1120, 1122, 1124, 1126 is coupled, but is not limited to those examples described above. Other methods or components may be used to provide the conductive pathway.

Similar to the coiled conductors 920, 922, 924, 926, the coiled conductors 1120, 1122, 1124, 1126 are held sufficiently close together by the outer jacket 1106 that the coiled conductors 1120, 1122, 1124, 1126 cancel out or significantly reduce RF-induced current in the coiled conductors 1120, 1124, 1126, 1128, as described above.

One difference between the embodiments of the lead assembly 102 shown in Figures 9 and 11 is the addition of an insulating sleeve 1156. The insulating sleeve 1156 is a dielectric tubular body that encircles the center axis 306 along at least a portion of the length of the body 300. As shown in Figure 11, the insulating sleeve 1156 may only be provided in the portions of the body 300 where multiple layers of the coiled conductors 1120, 1122, 1124, 1126 are provided. For example, the insulating sleeve 1156 may only extend to the electrode 324. Alternatively, the insulating sleeve 1156 may extend a greater length within the body 300. The insulating sleeve 1156 may be held within the outer jacket 1106 similar to the coiled conductors 1120, 1122, 1124, 1126. The insulating sleeve 1156 is an additional, separate body that is held within the outer jacket 1106. Alternatively, the insulating sleeve 1156 may represent a portion of the outer jacket 1106 that separates the different layers of coiled conductors 1120, 1122, 1124, 1126. The insulating sleeve 1156 may include or be formed from electrically insulative materials, such as polyurethane, silicone, a mixture of polyurethane and silicon (such as Optim™), ETFE, or PTFE, for example.

The insulating sleeve 1156 separates the layers of coiled conductors 1120, 1122, 1124, 1126 from each other in radial direction that extend outward from the center axis 306. As shown in Figure 11, the insulating sleeve 1156 provides an additional dielectric layer between an inner layer of the coiled conductors 1124, 1126 and an outer layer of the coiled conductors 1120, 1122. The insulating sleeve 1156 provides a dielectric layer between the inner and outer layers that is in addition to the insulative layers 1148, 1150, 1152, 1154. While only a single insulating sleeve 1156 is shown between two layers of the coiled conductors 1120, 1122, 1124, 1126, alternatively two or more insulating sleeves 1156 may be provided, with each insulating sleeve 1156 provided between different layers of coiled conductors 1120, 1122, 1124, 1126. For example, where the coiled conductors 1120, 1122, 1124, 1126 are arranged in three or more layers in the outer jacket 1106, a different and discrete insulating sleeve 1156 may be provided between adjacent layers of coiled conductors 1120, 1122, 1124, 1126.

The insulating sleeve 1156 prevents a safeguard against the coiled conductors 1120, 1122, 1124, 1126 in the layers from electrically contacting each other if the insulative layers 1148, 1150, 1152, 1154 are removed or separated from the coiled conductors 1120, 1122, 1124, 1126. For example, during flexing or bending of the body 300, one or more of the insulative layers 1148, 1150, 1152, 1154 may be removed from the corresponding underlying coiled conductor 1120, 1122, 1124, 1126. The insulating sleeve 1156 prevents one or more of the coiled conductors 1120, 1122 in the outer layer from electrically contacting and shorting with the coiled conductors 1124, 1126 in the inner layer. In another embodiment,

At least certain embodiments of the presently described subject matter seek to reduce or eliminate RF-induced current in conductors of a lead assembly. The application of certain inventive concepts described herein may enhance a heating reduction performance of self resonant RF chokes. For example, reducing the RF-induced current in the conductors of a lead assembly can reduce the amount or degree that the electrodes are heated by the RF-induced current.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings. Also, it is to be understood that phraseology and terminology used herein with reference to device or element orientation (such as, for example, terms like "central," "upper," "lower," "front," "rear," "distal," "proximal," and the like) are only used to simplify description of one or more embodiments described herein, and do not alone indicate or imply that the device or element referred to must have a particular orientation. In addition, terms such as "outer" and "inner" are used herein for purposes of description and are not intended to indicate or imply relative importance or significance.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the presently described subject matter without departing from its scope. While the dimensions, types of materials and coatings described herein are intended to define the parameters of the disclosed subject matter, they are by no means limiting and are exemplary embodiments. Many other embodiments will be apparent to those of skill in the art upon reviewing the above description. The scope of the invention should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects. Further, the limitations of the following claims are not written in means - plus-function format and are not intended to be interpreted based on 35 U.S.C. § 112, sixth paragraph, unless and until such claim limitations expressly use the phrase "means for" followed by a statement of function void of further structure.

### Examples are set out in the following list of numbered clauses.

1. An implantable lead assembly comprising:
an elongated outer jacket extending along a center axis, the outer jacket having a body that is configured to be implanted in a patient;
first and second coiled conductors molded within the body of the outer jacket, the first and second coiled conductors extending along the center axis and wound around the center axis, the first coiled conductor radially disposed farther from the center axis than the second coiled conductor; and
first and second electrodes coupled with the outer jacket, the first electrode electrically coupled to the first coiled conductor and the second electrode electrically coupled to the second coiled conductor.

2. The lead assembly of clause 1, wherein the first and second coiled conductors are enclosed in insulative layers that engage each other while electrically separating the first and second coiled conductors.

3. The lead assembly of clause 1, further comprising a third coiled conductor disposed within the outer jacket and a third electrode coupled with the outer jacket and electrically coupled with the third coiled conductor, the first and third coiled conductors radially disposed a common distance from the center axis.

4. The lead assembly of clause 1, further comprising third and fourth coiled conductors disposed within the outer jacket and third and fourth electrode coupled with the outer jacket and electrically coupled with the third and fourth coiled conductors, respectively, the first and third coiled conductors radially disposed a common distance from the center axis and the second and fourth coiled conductors radially disposed a different common distance from the center axis.

5. The lead assembly of clause 1, further comprising an inner liner disposed within the outer jacket with the first and second coiled conductors disposed between the outer jacket and the inner liner.

6. The lead assembly of clause 1, wherein the inner liner extends along and is radially separated from the center axis to define a lumen that extends along the center axis.

7. The lead assembly of clause 1, wherein the first and second conductors include interface ends configured to mate with a connector of an implantable medical device that supplies stimulus pulses to a heart of the patient or senses cardiac signals of the heart through the first and second coiled conductors and the first and second electrodes.

8. The lead assembly of clause 1, wherein the first and second conductors include interface ends configured to mate with a connector of a neurologic stimulation device that supplies stimulus pulses to a nervous system of the patient through the first and second coiled conductors and the first and second electrodes.

9. A method for providing an implantable lead assembly, the method comprising:
providing first and second coiled conductors that wrap around a center axis, the first and second coiled conductors enclosed in insulative layers that engage each other while electrically separating the first and second coiled conductors,
molding an elongated outer jacket around the first and second coiled conductors such that the first and second coiled conductors are encapsulated in a body of the outer jacket; and
electrically coupling first and second electrodes with the first and second coiled conductors, respectively.

10. The method of clause 9, wherein the providing the first and second coiled conductors includes providing the first coiled conductor around the center axis at a greater radial distance from the center axis than the second coiled conductor is wrapped around the center axis.

11. The method of clause 9, wherein the providing the first and second coiled conductors includes providing the first and second coiled conductors at a common radial distance from the center axis within the outer jacket.

12. The method of clause 11, wherein the providing the first and second coiled conductors includes providing a third coiled conductor that wraps around the center axis a different radial distance from the center axis than the first and second coiled conductors, and the coupling operation includes electrically coupling a third electrode with the third coiled conductor.

13. The method of clause 11, wherein the providing the first and second coiled conductors includes providing third and fourth coiled conductors that are wrapped around the center axis a different common radial distance than the first and second coiled conductors, and the coupling operation includes electrically coupling third and fourth electrodes with the third and fourth coiled conductors, respectively.

## Claims

1. An implantable lead assembly comprising:
an outer jacket elongated along a center axis, the outer jacket having a body that radially extends between opposite interior and exterior surfaces, the interior surface defining an elongated lumen;
first and second coiled conductors held within the body of the outer jacket between the interior and exterior surfaces of the outer jacket, the first and second coiled conductors wrapped around the lumen and the center axis; and
first and second electrodes coupled with the outer jacket, the first electrode electrically coupled to the first coiled conductor and the second electrode electrically coupled to the second coiled conductor in order to at least one of deliver stimulus pulses or sense electrical activity.

2. The lead assembly of claim 1, wherein the first and second coiled conductors are molded within the outer jacket.

3. The lead assembly of claim 1, wherein the first and second coiled conductors are disposed within insulative layers that electrically separate the first and second coiled conductors from each other, the outer jacket holding the insulative layer of the first coiled conductor against the insulative layer of the second coiled conductor within the outer jacket.

4. The lead assembly of claim 3, wherein the insulative layers of the first and second coiled conductors engage each other through a plurality of turns of the first and second coiled conductors around the lumen.

5. The lead assembly of claim 1, wherein the first and second coiled conductors are arranged in inner and outer layers with the first coiled conductor disposed in the outer layer and the second coiled conductor in the inner layer, the outer layer radially disposed farther from the center axis than the second coiled conductor.

6. The lead assembly of claim 5, further comprising one or more additional coiled conductors arranged in additional layers that are separate from the inner and outer layers, the additional layers disposed different radial distances from the center axis than the inner and outer layers.

7. The lead assembly of claim 5, further comprising an insulating sleeve disposed between the inner and outer layers to electrically separate the first and second coiled conductors from each other along radial directions.

8. The lead assembly of claim 1, wherein the first and second coiled conductors are arranged in a single layer disposed a common radial distance from the center axis, further comprising a third coiled conductor held within the outer jacket and a third electrode coupled with the outer jacket and electrically coupled with the third coiled conductor, the third coiled conductor radially disposed a different distance from the center axis than the first and second coiled conductors.

9. The lead assembly of claim 1, wherein the first and second coiled conductors are arranged in a single layer disposed a common radial distance from the center axis, further comprising third and fourth coiled conductors held within the outer jacket and third and fourth electrode coupled with the outer jacket and electrically coupled with the third and fourth coiled conductors, respectively, the third and fourth coiled conductors radially disposed a different common distance from the center axis than the first and second coiled conductors.

10. The lead assembly of claim 1, further comprising an inner liner disposed within the lumen and elongated along the center axis, the first and second coiled conductors disposed between the outer jacket and the inner liner.

11. The lead assembly of claim 1, wherein the first and second conductors include interface ends configured to mate with a connector of an implantable medical device that supplies stimulus pulses to a heart of the patient or senses cardiac signals of the heart through the first and second coiled conductors and the first and second electrodes.

12. The lead assembly of claim 1, wherein the first and second conductors include interface ends configured to mate with a connector of a neurologic stimulation device that supplies stimulus pulses to a nervous system of the patient through the first and second coiled conductors and the first and second electrodes.
